# EUROPÄISCHE PATENTSCHRIFT

(11) **EP 1 819 662 B1**
(45) Veröffentlichungstag und Bekanntmachung des Hinweises auf die Patenterteilung: **26.03.2008**
(21) Anmeldenummer: 05817984.7
(22) Anmeldetag: 29.11.2005
(51) Int. Cl.: C07C 233/03, C07C 231/24

(54) **VERFAHREN ZUR REINIGUNG VON POLAREN VINYLVERBINDUNGEN**
METHOD FOR PURIFYING POLAR VINYL COMPOUNDS
PROCEDE DE NETTOYAGE DE COMPOSES VINYLIQUES POLAIRES

(30) Priorität: 01.12.2004 DE 102004058071
(43) Veröffentlichungstag der Anmeldung: 22.08.2007
(73) Patentinhaber: BASF SE, 67056 Ludwigshafen (DE)
(72) Erfinder: JUDAT, Bernd, 68163 Mannheim (DE); RAULS, Matthias, 67061 Ludwigshafen (DE); RÜBENACKER, Martin, 67122 Altrip (DE); WINTER, Manfred, 67596 Dittelsheim-Hessloch (DE)
(86) Internationale Anmeldenummer: PCT/EP2005/012733
(87) Internationale Veröffentlichungsnummer: WO 2006/058698

(56) Entgegenhaltungen:
- EP-A- 0 336 564
- EP-A- 0 644 180
- US-A- 3 424 791
- US-A- 5 710 284

## Beschreibung

Die vorliegende Erfindung betrifft ein Verfahren zur Reinigung von polaren Vinylverbindungen, insbesondere die Kristallisation von offenkettigen N-Vinylverbindungen.

Polare Vinylverbindungen im Sinne der vorliegenden Erfindung sind offenkettige monoethylenisch ungesättigte Monomere, die zusätzlich Stickstoff als Heteroatom enthalten.

Aus solchen Vinylverbindungen werden mittels Polymerisation Homo- und Copolymere hergestellt, die in den verschiedensten Bereichen, wie beispielsweise in der Kosmetik-und Pharmaindustrie sowie in der Papierindustrie Anwendung finden.

Vinylverbindungen sind aufgrund der Doppelbindung sehr reaktiv und neigen leicht zur unkontrollierten Polymerisation. Daher werden den Vinylverbindungen zur besseren Handhabung beispielsweise bei Lagerung und Transport Polymerisationsinhibitoren zugesetzt, die eine unkontrollierte Polymerisation verhindern sollen. Nachteilig daran ist, dass aus den Polymerisationsinhibitoren enthaltenden Vinylverbindungen ohne einen weiteren Reinigungsschritt keine hochmolekularen Homo- und Copolymere hergestellt werden können, da die Polymerisationsinhibitoren die Polymerisation steuern und auf diese Weise das Molekulargewicht der Polymere begrenzt wird.

Solche hochmolekularen Polymere, die keine Verunreinigungen wie Polymerisationsinhibitoren enthalten, sind jedoch für viele Anwendungsbereiche wünschenswert.

Daher werden für die Herstellung hochmolekularer Polymere hochreine Vinylmonomere benötigt, die jedoch aufgrund der oben beschriebenen Polymerisationsneigung schwer erhältlich sind.

EP 1 048 646 A1 beschreibt ein Verfahren zur kontinuierlichen Destillation von thermolabilen Monomeren wie N-Vinylverbindungen unter vermindertem Druck in Gegenwart von Formamid. Das nach diesem Verfahren erhältliche Produkt weist noch einen Anteil von weniger als 5 Gew.-% Formamid auf, so dass eine Polymerisation zu einem hochmolekularen Polymer nicht möglich ist.

Die US 6,033,530 offenbart ein Verfahren zur Reinigung von thermolabilen Monomeren wie N-Vinylformamid mit Hilfe einer heterogenen azeotropen Destillation in Gegenwart eines Destillationshilfsstoffs.

Eine andere Möglichkeit zur Herstellung hochreiner Vinylverbindungen ist die Entfernung der Verunreinigung über lonenaustauscherharze oder Aktivkohle. Die Regeneration dieser Komponenten in den damit gepackten Säulen muss jedoch in gewissen Zeitabständen durchgeführt werden, was eine großtechnische Anwendung erschwert.

In der japanischen Offenlegungsschrift JP-A 61-286069 ist ein extraktives Trennverfahren beschrieben, in dem Wasser und aromatische Kohlenwasserstoffe als Lösemittel verwendet werden. Nachteilig an diesem Verfahren ist, dass einige Vinylverbindungen wie beispielsweise N-Vinylcarbonsäureamide in Wasser instabil sind und zur Hydrolyse neigen.

Aus der EP 0 644 180 A1 ist ein Verfahren zur Herstellung von hochreinen polaren Vinylverbindungen bekannt, in welchem eine Kristallisation unter hohen Drücken (500 - 3000 atm) und Temperaturen (0-100°C) durchgeführt wird. Dabei wird die Kristallisation in zwei Schritten durchgeführt, in einem ersten Schritt wird die polare Vinylkomponente unter Druck kristallisiert. Die Kristalle werden von der verbliebenen flüssigen Phase getrennt. Diese flüssige Phase ist mit Verunreinigungen angereichert und wird in einem zweiten Schritt erneut kristallisiert. Das zweite Kristallisat wird der Rohvinylverbindung zugemischt, welche wiederum der ersten Kristallisation zugeführt wird. Nachteilig an diesem Verfahren sind die hohen Prozess- und Investitionskosten aufgrund der hohen Drücke.

Die deutsche Offenlegungsschrift DE 195 36 792 A1 beschreibt ein Verfahren zur Stofftrennung aus einem flüssigen Gemisch durch Kristallisation, in dem auf diejenigen Flächen, von denen aus während der Kristallisation Kristalle wachsen sollen, eine zweiphasige Impfschicht in Form einer Schmelze oder Lösung des zu trennenden Stoffgemisches mit darin bereits suspendierten Kristallen aufgebracht wird. Das Verfahren bezieht sich allgemein auf zur Trennung geeignete flüssige Gemische mit einem Schmelzpunkt zwischen -50°C bis +300 °C, insbesondere geeignet sind u.a. N-Vinylpyrrolidon, Naphthalin und Acrylsäure.

DE 195 36 859 A1 offenbart ein Verfahren zur Reinigung von N-Vinylpyrrolidon durch Kristallisation, wobei diejenigen Flächen des Kristallers, von denen aus die Kristalle wachsen sollen, mit einer Impfschicht aus N-Vinylpyrrolidon belegt werden.

Nachteilig an den in beiden deutschen Offenlegungsschriften DE 195 36 792 A1 und DE 195 36 859 A1 beschriebenen Verfahren ist die aufwendige Belegung der Kristallerflächen mit einer Impfschicht.

In vielen Anwendungsbereichen besteht ein großes Interesse an hochreinen offenkettigen N-Vinylverbindungen, insbesondere N-Vinylformamid, die keine Verunreinigungen wie Polymerisationsinhibitoren enthalten und aus denen hochmolekulare Homo- und Copolymere hergestellt werden können.

Der vorliegenden Erfindung lag die Aufgabe zugrunde, ein Verfahren zur Reinigung einer offenkettigen N-Vinylverbindung zu finden, dass die Nachteile der im Stand der Technik beschriebenen Verfahren vermeidet.

Die Aufgabe wurde gelöst durch ein Verfahren zur Reinigung einer offenkettigen N-Vinylverbindung durch Kristallisation in einem Kristaller, in dem die Kristallisation aus einer Schmelze einer offenkettigen N-Vinylverbindung enthaltenden Mischung bei einem Druck von 10⁻³ bis 400 bar durchgeführt wird.

Vorteilhaft im Vergleich zu dem im Stand der Technik beschriebenen Verfahren ist, dass das erfindungsgemäße Verfahren ohne den Einsatz von Lösemitteln auskommt, sowie unter moderaten Drücken und unter wirtschaftlichem Energieaufwand durchführbar ist.

Unter offenkettigen N-Vinylverbindungen im Sinne der vorliegenden Erfindung werden offenkettige monoethylenisch ungesättigter Vinylverbindungen, die zusätzlich Stickstoff als Heteroatom enthalten, verstanden. Dabei ist es unerheblich, in welcher Position der Stickstoff relativ zur Doppelbindung steht.

Das erfindungsgemäße Verfahren kann sowohl als Schichtkristallisation als auch als Suspensionskristallisation ausgeübt werden.

Der Druck bei der Kristallisation nach dem erfindungsgemäßen Verfahren beträgt zwischen 10⁻³ bis 400 bar, bevorzugt zwischen 10⁻² und 250 bar, besonders bevorzugt zwischen 10⁻¹ und 100 bar und insbesondere zwischen 10⁻¹ und 50 bar. Besonders vorteilhaft ist es, wenn man das erfindungsgemäße Verfahren bei Atmosphärendruck durchführt. Dabei sind die angegebenen Druckwerte nicht absolut zu sehen, selbstverständlich sind Schwankungen im Bereich von ± 250 mbar möglich.

Die Temperatur in der kristallisierenden Schmelze liegt im Bereich von 0,1 bis 40 K unter dem Schmelzpunkt der reinen Schmelze, bevorzugt im Bereich von 0,2 bis 20 K und besonders bevorzugt im Bereich von 0,5 bis 10 K unter dem Schmelzpunkt der reinen Schmelze.

Das erfindungsgemäße Verfahren geht von einer durch Kristallisation zu reinigenden offenkettigen N-Vinylverbindung enthaltenden Mischung aus, die im folgenden auch Roh-N-Vinylverbindung genannt wird. Neben der offenkettigen N-Vinylverbindung enthält die Roh-N-Vinylverbindung noch Polymerisationsinhibitoren und Nebenkomponenten, die beispielsweise aus der Synthese der offenkettigen N-Vinylverbindung kommen. Im folgenden werden diese Verbindungen zusammengefasst als Verunreinigungen bezeichnet.

Typische Nebenkomponenten solcher Art sind Aldehyde wie z. B. Acetaldehyd, Formaldehyd und Crotonaldehyd, aber auch andere Nebenkomponenten wie Edukte, Hilfsmittel und Lösemittel aus der Herstellung der offenkettigen N-Vinylverbindung sind möglich.

In der Regel sind als Polymerisationsinhibtoren N-Oxyle (Nitroxyl- oder N-Oxyl-Radikale, Verbindungen, die wenigstens eine >N-O. - Gruppe aufweisen) in der Roh-N-Vinylverbindung enthalten, z.B. 4-Hydroxy-2,2,6,6-tetramethyl-piperidin-N-oxyl, 4-Oxo-2,2,6,6-tetramethyl-piperidin-N-oxyl, 4-Methoxy-2,2,6,6-tetramethyl-piperidin-N-oxyl und 2,2,6,6-Tetramethyl-piperidin-N-oxyl. Selbstverständlich können in der Roh-N-Vinylverbindung auch andere zur Stabilisierung von ethylenisch ungesättigten Verbindungen einsetzbaren Polymerisationsinhibitoren enthalten sein. Als Stabilisatoren sind generell phenolische Verbindungen, die genannten N-Oxyle, aromatische Amine, Phenylendiamine, Imine, Sulfonamide, Oxime, Oximether, Hydroxylamine, Harnstoffderivate, phosphorhaltige Verbindungen, schwefelhaltige Verbindungen wie Phenothiazin, Komplexbildner und Metallsalze, sowie Gemische davon geeignet.

Die Roh-N-Vinylverbindung kann aus einem beliebigen Herstellungsverfahren der offenkettigen N-Vinylverbindung stammen. Als Roh-N-Vinylverbindung werden bevorzugt Produktströme verwendet, die bereits aus einer destillativen Reinigung kommen. Solche Produktströme werden für gewöhnlich dem Seitenabzug oder am Kopf der Destillationskolonne entnommen. Die destillative Reinigung von N-Vinylverbindungen ist beispielsweise in den zuvor genannten Schriften EP 1 048 646 A1, US 6,033,530 sowie in EP 0 231 901 A1 beschrieben.

Besonders bevorzugt wird als Roh-N-Vinylverbindung der Produktstrom aus der destillativen Reinigung verwendet, der aufgrund seines hohen Anteils an Verunreinigungen nicht für die Polymerisation geeignet ist. Dieser Produktstrom enthält in der Regel weniger als 40 Gew.%, bevorzugt weniger als 20 Gew.-%, besonders bevorzugt weniger als 10 Gew.-% und ganz besonders bevorzugt weniger als 5 Gew.-%, bezogen auf die Roh-N-Vinylverbindung, an Verunreinigungen, d.h., der Gehalt an offenkettiger N-Vinylverbindung beträgt in der Regel mindestens 60 Gew.-%, bevorzugt mindestens 80 Gew.-%, besonders bevorzugt mindestens 90 Gew.-% und ganz besonders bevorzugt mindestens 95 Gew.-%, bezogen auf die Roh-N-Vinylverbindung.

Die offenkettige N-Vinylverbindung wird ein- oder mehrfach, vorzugsweise ein- oder zweifach, kristallisiert, bis der gewünschte Reinheitsgrad erreicht ist. Vorzugsweise arbeitet man dabei nach dem Gegenstromprinzip, d.h. die Mutterlauge der jeweiligen Kristallisationsstufe wird der jeweils vorangehenden Kristallisationsstufe zugeführt. Gegebenenfalls werden noch weitere Reinigungsschritte durchgeführt.

Vorzugsweise führt man die Kristallisation in der jeweiligen Kristallisationsstufe so weit, dass wenigstens 5 Gew.%, vorzugsweise wenigstens 10 Gew.-% und besonders bevorzugt wenigstens 20 Gew.-% der offenkettigen N-Vinylverbindung auskristallisiert wird. Typischerweise wird in einer Kristallisationsstufe nicht mehr als 90-Gew.-%, vorzugsweise nicht mehr als 80 Gew.-%, insbesondere nicht mehr als 70 Gew.-% der in der jeweiligen Kristallisationsstufe eingesetzten offenkettigen N-Vinylverbindung auskristallisiert, um eine hinreichende Reinigungswirkung zu erzielen.

Der im erfindungsgemäßen Verfahren einsetzbare Kristaller unterliegt an sich keiner Beschränkung. Als besonders geeignet haben sich Kristaller erwiesen, deren Funktion auf der Bildung von Kristallen auf gekühlten Flächen beruht. Derartige Kristallisationsverfahren werden auch als Schichtkristallisation bezeichnet. Geeignete Apparate finden sich in den in DE 102 57 449 A1 auf Seite 4, Zeile 6 und 7, angegebenen Patentschriften.

In einer Ausführungsform des erfindungsgemäßen Verfahrens wird die offenkettige N-Vinylverbindung unter Kühlung kristallisiert. Bei dieser sogenannten Schichtkristallisation werden die Kristalle von der Mutterlauge getrennt und aufgeschmolzen.

Zur Schichtkristallisation wird die zu reinigende Roh-N-Vinylverbindung mit einer Kühlfläche, beispielsweise den gekühlten Flächen eines Wärmetauschers in Kontakt gebracht. Dabei kühlt man die Wärmetauscherflächen des Kristallers vorzugsweise auf Temperaturen, die bis zu 40°C unterhalb der Schmelztemperatur der offenkettigen N-Vinylverbindung liegen. Bei Erreichen des gewünschten Kristallisationsgrades wird der Abkühlvorgang beendet und die verbleibende Flüssigkeit (Mutterlauge) abgeführt, beispielsweise durch Abpumpen oder Abfließen. Die Reinheit der auf den Wärmetauscherflächen des Kristallers verbleibenden Kristalle der offenkettigen N-Vinylverbindung kann noch erhöht werden, indem durch partielles Abschmelzen höher verunreinigte Anteile der Kristalle verflüssigt werden (Schwitzen) und abgeführt werden. Zudem besteht die Möglichkeit, die Reinheit der Kristalle auf der Wärmetauscherfläche durch Waschen mit einer Waschflüssigkeit zu erhöhen. Als Waschflüssigkeiten sind beispielsweise das flüssige Reinprodukt, also die offenkettige N-Vinylverbindung mit gewünschter Endreinheit, welche durch Schmelzen der Kristalle erhalten wird, oder die flüssige Roh-N-Vinylverbindung geeignet. Dabei ist jedoch zu beachten, dass die Waschflüssigkeit eine höhere Reinheit aufweist als die Mutterlauge, aus der das Kristallisat abgetrennt wurde. Das Waschen oder Schwitzen wird weiter unten näher beschrieben und kann unter Umständen eine weitere Kristallisationsstufe einsparen.

Die Isolierung der gereinigten, kristallisierten offenkettigen N-Vinylverbindung erfolgt üblicherweise durch Aufschmelzen der kristallisierten offenkettigen N-Vinylverbindung, beispielsweise durch Erwärmen der Wärmetauscherflächen auf eine Temperatur oberhalb der Schmelztemperatur der offenkettigen N-Vinylverbindung und/oder durch Zufuhr einer Schmelze gereinigter offenkettiger N-Vinylverbindung. Hierbei fällt die gereinigte offenkettige N-Vinylverbindung als Schmelze an und wird als solche isoliert. Auch kann man die kristalline offenkettige N-Vinylverbindung in Wasser oder einem geeigneten Lösemittel lösen und die so erhaltene Lösung direkt in der nachfolgenden Polymerisation einsetzen.

Die zur Schichtkristallisation erforderliche Temperatur hängt vom Grad der Verunreinigung ab. Die Obergrenze ist naturgemäß die Temperatur, bei der sich die bereits kristallisierte offenkettige N-Vinylverbindung mit der in der Mutterlauge enthaltenen offenkettigen N-Vinylverbindung im Gleichgewicht befindet (Gleichgewichtstemperatur). Je nach Zusammensetzung der Roh-N-Vinylverbindung liegt die Gleichgewichtstemperatur im Bereich von 0,1 bis 40 K unterhalb der Gleichgewichtstemperatur der reinen N-Vinylverbindung. Bevorzugt liegt die Gleichgewichtstemperatur der Roh-N-Vinylverbindung im Bereich von 0,2 bis 20 K und besonders bevorzugt im Bereich von 0,5 bis 10 K unterhalb der Gleichgewichtstemperatur der reinen N-Vinylverbindung.

In einer Ausführungsform des Kristallisationsverfahrens führt man die Schichtkristallisation in Gegenwart von Impfkristallen durch.

Die Kristallisation an Kühlflächen kann als dynamisches oder statisches Verfahren durchgeführt werden. Dynamische Verfahren sind beispielsweise aus EP 0 616 998 A1 bekannt, statische beispielsweise aus US 3,597,164. Bei den dynamischen Kristallisationsverfahren wird das zu kristallisierende Rohprodukt in einer strömenden Bewegung gehalten. Dies kann durch eine erzwungene Strömung in voll durchströmten Wärmetauschern geschehen, wie in DE 26 06 364 A1 beschrieben, oder durch einen Rieselfilm auf eine gekühlte Wand, wie Kühlwalzen oder Kühlbänder. Bei der statischen Kristallisation findet ein Stoffaustausch in der flüssigen Phase nur durch freie Konvektion statt (ruhende Schmelze). Die Schichtkristallisation an Kühlflächen im dynamischen Verfahrensbetrieb ist in der vorliegenden Erfindung bevorzugt.

Die statische Schichtkristallisation wird bevorzugt mit einem Impfvorgang eingeleitet. In einer besonderen Ausgestaltung des Impfvorgangs wird auf den Kühlflächen die als Restfilm nach dem Abschmelzen verbleibende Flüssigkeit als Impfkristallisat an der Kühlfläche partiell oder vollständig ausgefroren und danach erneut eine Kristallisation durchgeführt. Das Ausfrieren von lmpfkristallisat kann auch dadurch erfolgen, dass vor der Kristallisation lmpfkristallisat dadurch auf die Kühlfläche aufgebracht wird, dass die Kühlfläche in einem separaten Schritt mit einer, bezogen auf das zu trennende flüssige Stoffgemisch, reineren Schmelze der Roh-N-Vinylverbindung in Kontakt gebracht wird, anschließend davon abgetrennt wird und dann durch Abkühlen ein entsprechendes Impfkristallisat gebildet wird. Auch hierbei wird der auf den Kühlflächen verbleibende Restfilm durch Temperaturabsenkung an den Flächen partiell oder vollständig ausgefroren. Auch kann die Kühlfläche zur Erzeugung einer Impfkristallschicht mit einer kristallhaltigen Suspension der Roh-N-Vinylverbindung in Kontakt gebracht werden, um nach Entfernen der Suspension durch Abkühlung der Kühlfläche eine Impfkristallschicht auf derselben zu erhalten. Ebenso kann ein Impfen durch Zugabe von Kristallen als Feststoff oder als Suspension zur Schmelze der Roh-N-Vinylverbindung erreicht werden, wobei sich die Schmelze hierbei auf einer Temperatur nahe oder unter der Lösetemperatur befindet. Ein Impfen kann auch erreicht werden durch Erzeugung und/oder Erhaltung einer Kristallschicht auf einer lokal begrenzten, separat gekühlten Kühlfläche (sog. cold spot). Alternativ kann auch durch Zugabe eines Kühlmittes (z.B. Trockeneis) direkt gekühlt werden.

Die Kristallisation an Kühlflächen wird vorzugsweise einstufig durchgeführt, d.h. die erforderlich Endreinheit der offenkettigen N-Vinylverbindung wird bereits nach einer Kristallisationsstufe erreicht. Die Reinheit kann weiter erhöht werden, in dem die Kristallisation mehrstufig als sog. fraktionierte Kristallisation durchgeführt wird. Durch wiederholte Kristallisation der jeweils entstehenden reinen Fraktionen kann die gewünschte Endreinheit der offenkettigen N-Vinylverbindung eingestellt werden.

Die fraktionierte Kristallisation kann auch bei anderen geeigneten Kristallisationsverfahren, wie etwa der Suspensionskristallisation, angewendet werden.

Die Suspensionskristallisation kann als Alternative zur Schichtkristallisation durchgeführt werden. Bei der Suspensionskristallisation wird durch Kühlung des Rohproduktes, hier also der Roh-N-Vinylverbindung, eine Kristallsuspension in einer an Verunreinigungen angereicherten Schmelze erzeugt. Die Kristalle sind dispers in der Flüssigphase (Mutterlauge) verteilt und können dabei unmittelbar in der Suspension (Schmelze) wachsen oder sich als Schicht auf einer gekühlten Wand abscheiden. Von dieser werden die Kristalle anschließend bei Erreichen eines gewünschten Kristallgehaltes, der üblicherweise bei 5 bis 40 Gew.-% liegt, abgekratzt und in der Restschmelze suspendiert. Die Kristallsuspension wird vorzugsweise während des Verfahrens bewegt, wozu insbesondere umgepumpt oder gerührt wird. Dies ist aufgrund der hohen Feststoffdichten bei der Suspensionskristallisation und der großen Temperaturgradienten, welche zur Verkrustung der Wärmeübertragerflächen führen können, erforderlich. Neben der in der Lösungskristallisation üblichen Rührbehälter kommen auch andere Apparate, wie beispielsweise der Kratzkühler zum Einsatz. Dabei wird die entstehende Kristallschicht in einem innen durchflossenen und von außen gekühlten Doppelmantelrohr erzeugt und durch langsam rotierende Kratzelemente abgetragen und in die Schmelze zurückbefördert. Die Kristalle können anschließend eine Wachstumszone durchlaufen, in der sie im Falle einer Übersättigung weiterwachsen können. Ein anderer häufig verwendeter Apparat ist der Kühlscheibenkristaller. Hierbei entstehen die Kristalle an gekühlten Scheiben, die in die Schmelze eintauchen und kontinuierlich mit Hilfe von Schabern abgewischt werden. Neben diesen Suspensionskristallisationsverfahren mit indirekter Kühlung über Wärmeaustauschelemente lässt sich die Kühlung der Suspension auch direkt über die Einleitung eines Kühlmittels (z.B. von kalten Gasen oder Flüssigkeiten oder verdampfenden Flüssigkeiten) realisieren.

Die Suspensionskristallisation wird bevorzugt mit einem Impfvorgang eingeleitet. Ein Impfen kann durch Zugabe von Kristallen als Feststoff oder als Suspension zur Schmelze der Roh-N-Vinylverbindung erreicht werden, wobei sich dann die Schmelze zum Zugabezeitpunkt auf einer Temperatur nahe oder unter der Lösetemperatur befindet. Die zugegebenen Kristalle können speziell behandelt werden, z.B. zerkleinert und/oder gewaschen werden. Ein Impfen kann auch erreicht werden durch Erzeugung und/oder Erhaltung einer Kristallschicht auf einer lokal begrenzten, separat gekühlten Kühlfläche (sog. cold spot). Impfkristalle können auch von einer solchen separat gekühlten Fläche entfernt (z.B. mechanisch, durch Strömungskräfte oder durch Ultraschall) und in die Schmelze der Roh-N-Vinylverbindung eingetragen werden. Alternativ kann auch durch Zugabe eines Kühlmittels (z.B. Trockeneis) direkt gekühlt werden.

Eine angeimpfte Betriebsweise der Kristallisation kann auch dadurch erreicht werden, dass man die flüssige Schmelze zunächst stark abkühlt, bis spontan oder unter Anwendung eines zuvor beschriebenen Impfvorgangs eine Kristallbildung einsetzt, dann die Temperatur der Suspension wieder anhebt, um einen großen Teil des so gebildeten Kristallisats aufzuschmelzen, und dann in Anwesenheit des verbleibenden Restkristallisats (Impfkristalle) langsamer und gezielt abkühlt, um die gewünschte Suspension zu erzeugen.

Die Suspensionskristallisation kann kontinuierlich oder diskontinuierlich, vorzugsweise kontinuierlich, betrieben werden.

Zur Abtrennung der Flüssigphase (Mutterlauge) von einer durch Suspensionskristallisation auskristallisierten offenkettigen N-Vinylverbindung eignen sich alle bekannten Verfahren der Fest-Flüssig-Trennung, beispielsweise mittels einer Zentrifuge oder Filtration. Dem Zentrifugieren oder Filtrieren kann eine Voreindickung der Suspension, beispielsweise durch Hydrozyklone, vorgeschaltet sein. Die Filtration kann diskontinuierlich oder kontinuierlich unter Druck oder vermindertem Druck erfolgen. Bei Verwendung von Filternutschen können diese ein Rührwerk aufweisen.

Während und/oder nach der Fest-Flüssig-Trennung können weitere Verfahrensschritte, beispielsweise Waschen und Schwitzen, zur Steigerung der Reinheit der Kristalle bzw. des Kristallkuchens erfolgen. Beim Waschen liegt die Waschflüssigkeitsmenge vorzugsweise zwischen 5 und 500 g, bevorzugt zwischen 10 und 300 g, besonders bevorzugt zwischen 15 und 50 g Waschflüssigkeit pro 100 g Kristallisat. Als Waschflüssigkeiten sind beispielsweise das flüssige Reinprodukt, also die offenkettige N-Vinylverbindung mit gewünschter Endreinheit, welche durch Schmelzen der Kristalle erhalten wird, oder die flüssige Roh-N-Vinylverbindung geeignet. Dabei ist jedoch zu beachten, dass die Waschflüssigkeit eine höhere Reinheit aufweist als die Mutterlauge, aus der das Kristallisat abgetrennt wurde. Das Waschen oder Schwitzen kann unter Umständen eine weitere Kristallisationsstufe einsparen.

Das Waschen kann in hierfür üblichen Apparaten erfolgen. Vorteilhafterweise werden Waschkolonnen, in denen die Abtrennung der Mutterlauge und das Waschen in einem Schritt erfolgen, ein- oder mehrstufig betriebene Zentrifugen sowie Filternutschen oder Bandfilter verwendet. Sowohl auf Zentrifugen als auch auf Bandfiltern kann das Waschen ein- oder mehrstufig durchgeführt werden. Wird bereits die Kristallisation in einem statischen Kristaller betrieben, so wird das Waschen vorteilhafterweise im Kristaller selbst durchgeführt.

Beim Schwitzen handelt es sich um ein lokales Abschmelzen verunreinigter Bereiche der Kristalle. Hierzu wird die Temperatur der Kristallschicht etwas angehoben, beispielsweise um 0,5 bis 5°C oberhalb der Schmelztemperatur, wobei bevorzugt die höher verunreinigten Bereiche der Kristallschicht abschmelzen und so eine zusätzliche Reinigungswirkung erzielt wird. Das Schwitzprodukt wird dann der Mutterlauge zugeführt und mit dieser weiter verarbeitet. Vorteilhafterweise beträgt die Schwitzmenge zwischen 1 und 35 g, bevorzugt zwischen 10 und 30 g abgeschmolzenes Kristallisat pro 100 g Kristallisat vor dem Schwitzen. Wird bereits die Kristallisation in einem statischen Kristaller betrieben, so wird das Schwitzen vorteilhafterweise im Kristaller selbst durchgeführt.

Auch die Durchführung einer Kombination aus Waschen und Schwitzen in einem Apparat ist zur Steigerung der Reinheit der Kristalle bzw. des Kristallkuchens geeignet.

Die durch Kristallisation erzeugte offenkettige N-Vinylverbindung hat eine Reinheit von > 98 %, bevorzugt ≥ 99 %, besonders bevorzugt ≥99,5 % und insbesondere ≥ 99,9 %.

Die vorliegende Erfindung betrifft ein Verfahren zur Reinigung offenkettiger monoethylenisch ungesättigter Vinylverbindungen, die zusätzlich Stickstoff als Heteroatom enthalten. Dabei ist es unerheblich, in welcher Position der Stickstoff relativ zur Doppelbindung steht. Zu diesen N-Vinylverbindungen zählen beispielsweise N-Vinylcarbonsäureamide.

Allgemein lassen sich die offenkettigen N-Vinylverbindungen z.B. mit Hilfe der folgenden Formel beschreiben:

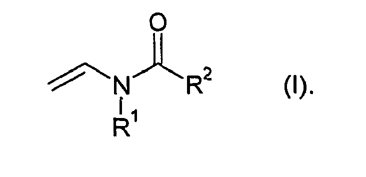

Darin können R¹ und R² gleich oder verschieden sein und für Wasserstoff und C₁- bis C₆-Alkyl stehen. Monomere dieser Art sind z.B. N-Vinylformamid (R¹ = R² = H in der Formel (I)), N-Vinyl-N-methylformamid, N-Vinylacetamid, N-Vinyl-N-methylacetamid, N-Vinyl-N-ethylacetamid, N-Vinyl-N-methylpropionamid und N-Vinylpropionamid. Das erfindungsgemäße Verfahren eignet sich insbesondere zur Herstellung von hochreinem N-Vinylfomamid.

Ebenfalls erfindungsgemäß zu reinigende Monomere sind solche der Formel (II): 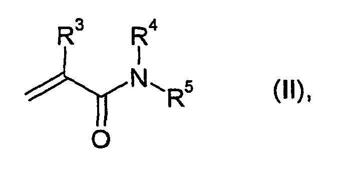 worin R³, R⁴ und R⁵ gleich oder verschieden sein. R³ kann Wasserstoff oder C₁- bis C₆-Alkyl sein, R⁴ und R⁵ können unabhängig Wasserstoff oder eine C₁- bis C₆-Alkyl, bevorzugt C₂-C₄-Alkyl, die gegebenenfalls mit einer Hydroxyl-, einer Dialkylamino-, einer Sulfat- oder einer qauternären Ammoniumgruppe substituiert ist, sein. Monomere dieser Art sind beispielsweise Acrylamide wie N-Methylacrylamid, N-Ethylacrylamid, N-lsopropylacrylamid, Monomethylolacrylamid, Diacetonacrylamid, N,N-Dimethylacrylamid, N,N-Diethylacrylamid, N,N-Methylenbisacrylamid, 2-Acrylamido-2-methylpropansulfonsäure oder deren Natriumsalz und N-Methylolacrylamid sowie von den genannten Verbindungen die Methacrylamid-Derivate.

Nach dem erfindungsgemäßen Verfahren wird besonders vorteilhaft N-Vinylformamid gereinigt. Der Vorteil gegenüber bekannten Verfahren liegt vor allem darin, dass man Monomerqualitäten erhält, die zu besonders hochmolekularen Polymeren verarbeitet werden könne. So erhält man beispielsweise aus erfindungsgemäß kristallisiertem N-Vinylformamid nach dem Verfahren der Öl-in-Wasser-Emulsionspolymerisation Poly-N-vinylformamide mit K-Werten nach Fikentscher oberhalb von 230 (gemessen in 5 gew.-%iger wässriger Kochsalzlösung bei 25 °C, pH 7 und einer Polymerkonzentration von 0,1 Gew.-%). Die Herstellung von so hochmolekularen Poly-N-vinylformamiden ist schwierig, weil bereits Verunreinigungen, die im Bereich von einigen ppm liegen, die Polymerisation von N-Vinylformamid erheblich beeinflussen.

Gegenstand der vorliegenden Anmeldung ist daher ebenfalls die Herstellung von hochmolekularen Homo- und Copolymeren aus den offenkettigen N-Vinylverbindungen, insbesondere von Poly-N-vinylformamid, wobei die K-Werte bevorzugt oberhalb von 230 liegen.

Weiterhin ist die Verwendung der hochmolekularen Homo- und Copolymerisate in der Papier-, Pharma und Kosmetikindustrie Gegenstand der vorliegenden Anmeldung.

Die Erfindung soll durch das folgende Beispiel näher erläutert werden, ohne sie jedoch darauf zu beschränken.

Der K-Wert wurde nach der oben beschriebenen Methode nach H. Fikentscher, Cellulose-Chemie, Band 13, 58-64 und 71-74 (1932), bestimmt (gemessen in 5 gew.-%.iger wässriger Kochsalzlösung bei 25°C, pH 7 und einer Polymerkonzentration von 0,1 Gew.-%).

Die Prozentangaben im Beispiel beziehen sich, falls nichts anders angegeben ist, auf das Gewicht.

Bei der anschließenden Polymerisation von hochreinem N-Vinylformamid zu hochmolekularem Polyvinylformamid wurden folgende Emulgatoren verwendet:

Span^{®} 80: Sorbitanmonooleat der Firma ICI

Hypermer^{®} B246: Polyester-Polyethylenoxid-Polyester-Blockcopolymerisat mit einer Molmasse > 1.000 g/mol, das durch Umsetzung von kondensierter 12-Hydroxystearinsäure mit Polyethylenoxid gemäß der Lehre der EP 0 000 424 hergestellt wird.

### Beispiele

### Beispiel 1

### Herstellung von hochreinem N-Vinylformamid (statische Schichtkristallisation)

3070 g einer Schmelze von N-Vinylformamid mit einer Reinheit von etwa 97,5 Gew.-% mit Verunreinigungen an Formamid, Crotonaldehyd sowie weiteren Verunreinigungen wurden bei Atmosphärendruck in ein senkrecht angeordnetes 3-Liter-Doppelmantelrohr mit einem Durchmesser von 50 mm eingefüllt, auf -11°C abgekühlt und durch Zugabe einer kleinen Menge an Trockeneis zur Kristallisation gebracht. Durch Aufheizen auf - 9,5°C wurde ein großer Teil des entstandenen Kristallisates wieder aufgelöst, so dass nur wenige Impfkristalle in der Schmelze verblieben. Danach wurde mit einer Abkühlrate von 0,3 K/h bis auf eine Temperatur von -12,5°C in 10 Stunden abgekühlt, bis etwa 1880 g ausgefroren waren. Bei dieser Temperatur ließ man die Restschmelze in ein Gefäß ablaufen. Das Kristallisat wurde anschließend mit einer Heizgeschwindigkeit von 0,5 K/h bis zu einer Temperatur von -8°C teilweise wieder aufgeschmolzen (Schwitzen). Die abgeschmolzene Masse ließ man ebenfalls aus dem Rohkristaller in ein Gefäß ablaufen, so dass eine Masse von 1490 g Kristallisat im Kristaller verblieb. Um dieses gereinigte Produkt aus dem Kristalier zu entfernen, wurde die Temperatur weiter erhöht, vollständig wieder aufgeschmolzen und ein separates Gefäß abgelassen. Die Reinheit des abgeschmolzenen Kristallisates wurde zu >99,5 Gew.-% bestimmt.

Polymerisation von hochreinem N-Vinylformamid zu hochmolekularem Poly-N-vinylformamid

In einem 21 fassenden Polymerisationsreaktor, der mit Ankerrührer, Rückflusskühler, Thermometer, Stickstoffeinlass versehen ist, werden unter Rühren folgende Substanzen vorgelegt: 256,1 g eines Kohlenwasserstoffgemisches vom Siedebereich 192 bis 254°C (Shellsol^{®} D70), 9 g Span^{®} 80 und 3 g Hypermer^{®} B246. Dazu wird eine Lösung von 5,88 g 75 %iger Phosphorsäure, 7,92 g 25 %iger Natronlauge und 303 g des hochreinem frisch kristallisierten N-Vinylformamids in 383 g Wasser mit einem pH-Wert von 6,5 zugesetzt. Der Behälterinhalt wird 1 Stunde bei einer Rührgeschwindigkeit von 350 Upm unter Einleiten von 10 l/h Stickstoff emulgiert. Anschließend werden bei einer Rührgeschwindigkeit von 250 Upm 0,45 g 2,2'-Azobis(4-methoxy-2,4-dimethylvaleronitril) und 0,15 g 2,2'-Azobis(2,4-dimethylvaleronitril) suspendiert in 10 g Kohlenwasserstoffgemisch (Shellsol^{®} D70) über einen Zeitraum von 6 Stunden zugesetzt. Es wurden insgesamt 15 Stunden bei 30-31 °C gerührt, anschließend wurde noch 4 Stunden bei 40 °C auspolymerisiert.

Der K-Wert nach Fikentscher betrug 235. Crotonaldehyd konnte nicht mehr nachgewiesen werden. Der Anteil an Formamid war auf etwa ein Drittel reduziert.

### Beispiel 2

### Herstellung von hochreinem N-Vinylformamid (Suspensionskristallisation)

1800 g einer Rohlösung N-Vinylformamid mit einer Verunreinigung von 0,69 % Formamid sowie weiterer Verunreinigungen im ppm-Bereich wurden bei Atmosphärendruck in einem senkrecht angeordneten 1,5-Liter-Rohrkristallisator mit einem wandgängigen Wendelrührer eingefüllt und von -8,3°C bis -10,3°C mit 0,5 K/h abgekühlt. Bei der Abkühlung entstanden in der Schmelze Kristalle, die durch das Rührorgan in Suspension gehalten wurden. Nach Erreichen der Endtemperatur befanden sich im Kristallisator ca. 42 Gew.-% Feststoff. Der Krisllisatorinhalt wurde auf einer Siebbecherzentrifuge bei 2000 min⁻¹ innerhalb von 3 min. abgetrennt. Ein Teil des Kristallisats wurde mit einem Gaschromatograph analysiert. Es wurden 0,08 % Formamid gefunden.

Polymerisation von hochreinem N-Vinylformamid zu hochmolekularem Poly-N-Vinylformamid

Ein anderer Teil des Kristallisats wurde zu hochmolekularem Poly-N-Vinylformamid wie in Beispiel 1 beschrieben polymerisiert. Der K-Wert nach Fikentscher betrug 228.

## Patentansprüche

1. Verfahren zur Reinigung einer offenkettigen N-Vinylverbindung durch Kristallisation in einem Kristaller, **dadurch gekennzeichnet, dass** die Kristallisation aus einer Schmelze einer offenkettigen N-Vinylverbindung enthaltenden Mischung bei einem Druck von 10⁻³ bis 400 bar durchgeführt wird.

2. Verfahren nach Anspruch 1, **dadurch gekennzeichnet, dass** die Kristallisation bei einem Druck von 10⁻¹ bis 50 bar durchgeführt wird.

3. Verfahren nach den Ansprüchen 1 und 2, **dadurch gekennzeichnet, dass** die Kristallisation bei Atmosphärendruck durchgeführt wird.

4. Verfahren nach einem der Ansprüche 1 bis 3, **dadurch gekennzeichnet, dass** es sich um eine Schichtkristallisation handelt.

5. Verfahren nach einem der Ansprüche 1 bis 3, **dadurch gekennzeichnet, dass** es sich um eine Suspensionskristallisation handelt.

6. Verfahren nach einem der Ansprüche 1 bis 5, **dadurch gekennzeichnet, dass** die Kristallisation als fraktionierte Kristallisation durchgeführt wird.

7. Verfahren nach einem der Ansprüche 1 bis 6, **dadurch gekennzeichnet, dass** die Reinheit der Kristalle durch Waschen und/oder Schwitzen erhöht wird.

8. Verfahren nach einem der Ansprüche 1 bis 7, **dadurch gekennzeichnet, dass** die offenkettige N-Vinylverbindung enthaltende Mischung eine Roh-N-Vinylverbindung ist, die neben der offenkettigen N-Vinylverbindung noch Polymerisationsinhibitoren und Nebenkomponenten enthält.

9. Verfahren nach Anspruch 8, **dadurch gekennzeichnet, dass** der Gehalt an öffenkettiger N-Vinylverbindung in der Roh-N-Vinylverbindung mindestens 90 Gew.-% beträgt.

10. Verfahren nach einem der Ansprüche 1 bis 9, **dadurch gekennzeichnet, dass** die offenkettige N-Vinylverbindung ausgewählt ist aus den Verbindungen der allgemeinen Formel I 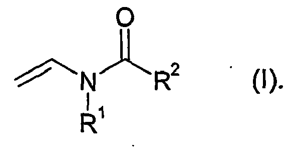 worin R¹ und R² gleich oder verschieden sein können und für Wasserstoff und C₁- bis C₆-Alkyl stehen,
und Verbindungen der allgemeinen Formel II 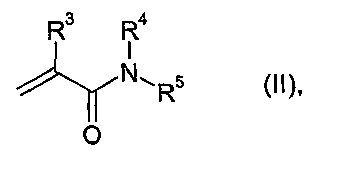 worin R³, R⁴ und R⁵ gleich oder verschieden sein können und R³ für Wasserstoff oder C₁- bis C₆-Alkyl steht und R⁴ und R⁵ unabhängig voneinander Wasserstoff oder eine C₁- bis C₆-Alkylgruppe, die gegebenenfalls mit einer Hydroxyl-, einer Dialkylamino-, einer Sulfat- oder einer qauternären Ammoniumgruppe substituiert ist, sein können.

11. Verfahren nach Anspruch 10, **dadurch gekennzeichnet, dass** es sich bei den Verbindungen der allgemeinen Formel I um N-Vinylformamid, N-Vinyl-N-methylformamid, N-Vinylacetamid, N-Vinyl-N-methylacetamid, N-Vinyl-N-ethylacetamid, N-Vinyl-N-methylpropionamid und N-Vinylpropionamid handelt.

12. Verfahren nach Anspruch 10, **dadurch gekennzeichnet, dass** es sich bei den Verbindungen der allgemeinen Formel II um Acrylamide wie N-Methylacrylamid, N-Ethylacrylamid, N-Isopropylacrylamid, Monomethylolacrylamid, Diacetonacrylamid, N,N-Dimethylacrylamid, N,N-Diethylacrylamid, N,N-Methylenbisacrylamid, 2-Acrylamido-2-methylpropansulfonsäure oder deren Natriumsalz und N-Methylolacrylamid sowie von den genannten Verbindungen die Methacrylamid-Derivate handelt

13. Verfahren nach Anspruch 11, **dadurch gekennzeichnet, dass** es sich bei der offenkettigen N-Vinylverbindung um N-Vinylfomamid handelt.

14. Verfahren zur Herstellung von hochmolekularen Homo- und Copolymeren, **dadurch gekennzeichnet, dass** diese aus den nach einem der Ansprüche 1 bis 13 hergestellten offenkettigen N-Vinylverbindungen synthetisiert werden.

15. Verfahren nach Anspruch 14, **dadurch gekennzeichnet, dass** es sich bei dem hochmolekularem Homo- und Copolymeren um Poly-N-vinylformamid mit einem K-Wert oberhalb von 230 handelt.

## Claims

1. A method of purifying an open-chain N-vinyl compound by crystallization in a crystallizer, which comprises crystallizing from a melt of a mixture comprising open-chain N-vinyl compound at a pressure of 10⁻³ to 400 bar.

2. The method according to claim 1, wherein crystallization is conducted at a pressure of 10⁻¹ to 50 bar.

3. The method according to claims 1 and 2, wherein crystallization is conducted at atmospheric pressure.

4. The method according to any one of claims 1 to 3, comprising layer crystallization.

5. The method according to any one of claims 1 to 3, comprising suspension crystallization.

6. The method according to any one of claims 1 to 5, wherein crystallization is conducted as fractional crystallization

7. The method according to any one of claims 1 to 6, wherein the crystal purity is raised by washing and/or sweating.

8. The method according to any one of claims 1 to 7, wherein the mixture comprising open-chain N-vinyl compound is a crude N-vinyl compound which besides the open-chain N-vinyl compound comprises polymerization inhibitors and secondary components.

9. The method according to claim 8, wherein the amount of open-chain N-vinyl compound in the crude N-vinyl compound is at least 90% by weight.

10. The method according to any one of claims 1 to 9, wherein the open-chain N-vinyl compound is selected from the compounds of the general formula 1 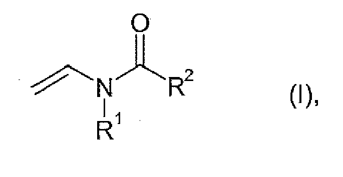 in which R¹ and R² can be identical or different and are hydrogen and C₁ to C₆ alkyl
and compounds of the general formula II 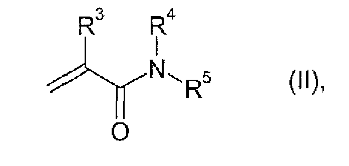 in which R³, R⁴ and R⁵ can be identical or different and R³ is hydrogen or C₁ to C₆ alkyl and R⁴ and R⁵ independently of one another can be hydrogen or a C₁ to C₆ alkyl group which is optionally substituted by a hydroxyl group, a dialkylamino group, a sulfate group or a quaternary ammonium group.

11. The method according to claim 10, wherein the compounds of the general formula I comprise N-vinylformamide, N-vinyl-N-methylformamide, N-vinylacetamide, N-vinyl-N-methylacetamide, N-vinyl-N-ethylacetamide, N-vinyl-N-methylpropionamide and N-vinylpropionamide.

12. The method according to claim 10, wherein the compounds of the general formula 11 comprise acrylamides such as N-methylacrylamide, N-ethylacrylamide, N-isopropylacrylarnide, monomethylolacrylamide, diacetoneacrylamide, N,N-dimethylacrylamide, N,N-diethylacryiamide, N,N-methylenebisacrylamide, 2-acrylamido-2-methylpropanesulfonic acid or its sodium salt and N-methylolacrylamide and also, of the compounds stated, the methacrylamide derivatives.

13. The method according to claim 11, wherein the open-chain N-vinyl compound is N-vinylformamide.

14. A process for preparing high molecular mass homopolymers and copolymers, which comprises synthesizing them from the open-chain N-vinyl compounds prepared according to any one of claims 1 to 13.

15. The process according to claim 14, wherein the high molecular mass homopolymers and copolymers comprise poly-N-vinylformamide having a K value of more than 230.

## Revendications

1. Procédé de purification d'un composé N-vinylique à chaîne ouverte par cristallisation dans un cristallisateur, **caractérisé en ce que** la cristallisation s'effectue à partir d'une fusion d'un mélange contenant le composé N-vinylique à chaîne ouverte à une pression de 10⁻³ à 400 bars.

2. Procédé selon la revendication 1, **caractérisé en ce que** la cristallisation a lieu à une pression de 10⁻¹ à 50 bars.

3. Procédé selon les revendications 1 et 2, **caractérisé en ce que** la cristallisation a lieu à pression atmosphérique.

4. Procédé selon l'une des revendications 1 à 3, **caractérisé en ce qu'**il s'agit d'une cristallisation en couche.

5. Procédé selon l'une des revendications 1 à 3, **caractérisé en ce qu'**il s'agit d'une cristallisation en suspension.

6. Procédé selon l'une des revendications 1 à 5, **caractérisé en ce que** la cristallisation se fait sous la forme d'une cristallisation fractionnée.

7. Procédé selon l'une des revendications 1 à 6, **caractérisé en ce que** la pureté des cristaux augmente par lavage et/ou suintage.

8. Procédé selon l'une des revendications 1 à 7, **caractérisé en ce que** le mélange contenant le composé N-vinylique à chaîne ouverte est un composé N-vinylique brut qui contient, à côté du composé N-vinylique à chaîne ouverte, encore des inhibiteurs de polymérisation et des composants secondaires.

9. Procédé selon la revendication 8, **caractérisé en ce que** la teneur en composé N-vinylique à chaîne ouverte dans le composé N-vinylique brut est d'au moins 90 % en poids.

10. Procédé selon l'une des revendications 1 à 9, **caractérisé en ce que** le composé N-vinylique à chaîne ouverte est choisi parmi les composés de formule générale I : 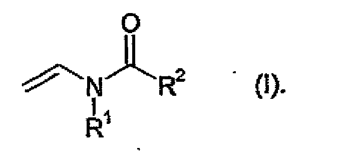 dans laquelle R¹ et R² peuvent être identiques ou différents et représenter l'hydrogène et un alkyle en C₁-C₆,
et des composés de formule générale II : 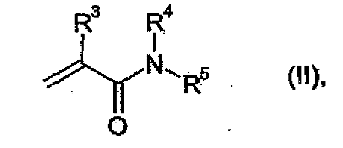 dans laquelle R³, R⁴ et R⁵ peuvent être identiques ou différents et R³ représente l'hydrogène ou un alkyle en C₁-C₆ et R⁴ et R⁵ peuvent être indépendamment l'un de l'autre l'hydrogène ou un groupe alkyle en C₁-C₆, qui est éventuellement substitué par un groupe hydroxyle, dialkylamino,
sulfate ou ammonium quaternaire.

11. Procédé selon la revendication 10, **caractérisé en ce qu'**il s'agit, pour les composés de formule générale I, de N-vinylformamide, de N-vinyl-N-méthylformamide, de N-vinylacétamide, de N-vinyl-N-méthylacétamide, de N-vinyl-N-éthylacétamide, de N-vinyl-N-méthylpropionamide et de N-vinylpropionamide.

12. Procédé selon la revendication 10, **caractérisé en ce que**, pour les composés de formule générale II, il s'agit d'acrylamides comme les N-méthylacrylamide, N-éthylacrylamide, N-isopropylacrylamide, monométhylolacrylamide, diacétonacrylamide, N,N-diméthylacrylamide, N,N-diéthylacrylamide, N,N-méthylènebisacrylamide, acide 2-acrylamido-2-méthylpropanesulfonique ou son sel de sodium et N-méthylolacrylamide ainsi que les dérivés de méthacrylamide des composés cités.

13. Procédé selon la revendication 11, **caractérisé en ce que** pour le composé N-vinylique à chaîne ouverte, il s'agit de N-vinylformamide.

14. Procédé de fabrication d'homo- et copolymères de poids moléculaire élevé, **caractérisé en ce que** ceux-ci sont synthétisés à partir des composés N-vinyliques à chaîne ouverte fabriqués selon l'une des revendications 1 à 13.

15. Procédé selon la revendication 14, **caractérisé en ce que**, pour l'homo- et copolymère de poids moléculaire élevé, il s'agit du poly-N-vinylformamide d'une valeur K supérieure à 230.
